(19) Europäisches Patentamt
European Patent Office
Office européen des brevets

(11) EP 4 663 706 A1

(12) **EUROPEAN PATENT APPLICATION**
published in accordance with Art. 153(4) EPC

(43) Date of publication:
**17.12.2025 Bulletin 2025/51**

(21) Application number: **24915498.0**

(22) Date of filing: **26.12.2024**

(51) International Patent Classification (IPC):
***C08L 101/00*** *(2006.01)* ***C08J 3/22*** *(2006.01)*
***C08J 5/18*** *(2006.01)* ***C08L 101/02*** *(2006.01)*

(52) Cooperative Patent Classification (CPC):
**B29B 7/48; B29C 48/40; B29C 48/57; C08J 3/22; C08J 5/18; C08L 29/04; C08L 67/04; C08L 101/00; C08L 101/02; G01N 33/44**

(86) International application number:
**PCT/JP2024/046223**

(87) International publication number:
**WO 2025/146809 (10.07.2025 Gazette 2025/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.01.2024 JP 2024000843**

(71) Applicant: **Daikin Industries, Ltd.**
**Osaka-shi, Osaka 530-0001 (JP)**

(72) Inventors:
- **SAKAMI, Kazuki**
  **Osaka-Shi, Osaka 530-0001 (JP)**
- **YAMATO, Takafumi**
  **Osaka-Shi, Osaka 530-0001 (JP)**
- **ITOH, Ryuo**
  **Osaka-Shi, Osaka 530-0001 (JP)**

(74) Representative: **Hoffmann Eitle**
**Patent- und Rechtsanwälte PartmbB**
**Arabellastraße 30**
**81925 München (DE)**

(54) **THERMOPLASTIC RESIN COMPOSITION, MOLDED BODY, PRODUCTION METHOD FOR THERMOPLASTIC RESIN COMPOSITION, AND EVALUATION METHOD FOR THERMOPLASTIC RESIN COMPOSITION**

(57) The disclosure aims to provide a thermoplastic resin composition having good processibility, a molded article, and a method for producing a thermoplastic resin composition. Provided is a thermoplastic resin composition containing: a resin (A); and a thermoplastic resin (B), the resin (A) containing a structural unit represented by the following formula 1 and having a dispersed particle size of 1 to 100 μm:

$$-X-(CR^1R^2)_n-Y-(CR^3R^4)_m-Z- \qquad \text{(formula 1).}$$

EP 4 663 706 A1

## Description

TECHNICAL FIELD

The disclosure relates to thermoplastic resin compositions, molded articles, methods for producing thermoplastic resin compositions, and methods for evaluating thermoplastic resin compositions.

BACKGROUND ART

In processing of melt-fabricable thermoplastic resins, rapid extrusion is required to achieve improved productivity and low cost. Still, melt-fabricable thermoplastic resin compositions inevitably have a critical shear rate and extrusion at a rate higher than this critical shear rate causes a rough surface called melt fracture, resulting in failure in providing a good molded article.

Techniques for improving the processibility of thermoplastic resins have been proposed. For example, Patent Literature 1 proposes a technique involving use of ethylene-vinyl alcohol and Patent Literature 2 proposes a technique involving use of a surfactant and polyethylene glycol. However, the effects thereof are insufficient.

CITATION LIST

- Patent Literature

Patent Literature 1: JP H01-215840 A
Patent Literature 2: US 2023/0031000 A1

SUMMARY OF INVENTION

- Technical Problem

The disclosure aims to provide a thermoplastic resin composition having good processibility, a molded article, and a method for producing a thermoplastic resin composition. The disclosure also aims to provide a method for evaluating a thermoplastic resin composition, which enables highly accurate evaluation of the dispersed particle size of the resin.

- Solution to Problem

The disclosure (1) relates to a thermoplastic resin composition containing: a resin (A); and a thermoplastic resin (B), the resin (A) containing a structural unit represented by the following formula 1 and having a dispersed particle size of 1 to 100 μm:

$$-X-(CR^1R^2)_n-Y-(CR^3R^4)_m-Z- \qquad \text{(formula 1)}$$

wherein X is a single bond or a divalent group optionally containing a functional group;

Y and Z are each independently a group constituted by at least one selected from the group consisting of a single bond, -O-, -C(=O)-, -C(=O)O-, -OC(=O)O-, -C(=NR')-, - C(=NR')O-, -OC(=NR')O-, -S-, -S(=O)-, -S(=O)O-, -OS(=O)O-, $-S(=O)_2-$, $-S(=O)_2O-$, $-OS(=O)_2O-$, -P(=O)-, -P(=O)O-, - OP(=O)O-, $-P(=O)_2-$, $-P(=O)_2O-$, $-OP(=O)_2O-$, -NR'-, and - C(OR')R'-, wherein R's at respective occurrences are each independently a hydrogen atom or a C1-C10 hydrocarbon group;
$R^1$, $R^2$, $R^3$, and $R^4$ are each independently a hydrogen atom or a C1-C10 hydrocarbon group; and
n and m are each independently an integer of 0 to 10,
with at least one of X, Y, or Z being -C(=O)-, - C(=O)O-, -OC(=O)O-, or -C(OR')R'-.

The disclosure (2) relates to the thermoplastic resin composition according to the disclosure (1), wherein the thermoplastic resin composition is substantially free from fluorine.

The disclosure (3) relates to the thermoplastic resin composition according to the disclosure (1) or (2), wherein, in the formula 1, X is a divalent group constituted by at least one selected from the group consisting of $X^1$ and $X^2$,

$X^1$ is a group constituted by at least one selected from the group consisting of -C(=O)-, -C(=NR')-, -S(=O)$_2$-, -NR'-, -CR'R'-, and -C(OR')R'-, wherein R's at respective occurrences are each independently a hydrogen atom or a C1-C10 hydrocarbon group; and

$X^2$ is a C1-C12 aromatic hydrocarbon group optionally containing a substituent.

The disclosure (4) relates to the thermoplastic resin composition according to the disclosure (3), wherein, in the formula 1, X is a divalent group containing at least one selected from the group consisting of -C(=O)-, -CR'R'-, and -C(OR')R'-, wherein R's at respective occurrences are each independently a hydrogen atom or a C1-C10 hydrocarbon group.

The disclosure (5) relates to the thermoplastic resin composition according to any one of the disclosures (1) to (4), wherein, in the formula 1, Y and Z are each independently a group constituted by at least one selected from the group consisting of a single bond, -O-, -C(=O)-, - C(=O)O-, -C(=NR')-, -C(=NR')O-, -S-, -S(=O)$_2$-, -S(=O)$_2$O-, - NR'-, and -C(OR')R'-, wherein R's at respective occurrences are each independently a hydrogen atom or a C1-C10 hydrocarbon group.

The disclosure (6) relates to the thermoplastic resin composition according to the disclosure (5), wherein, in the formula 1, Y and Z are each independently a group constituted by at least one selected from the group consisting of a single bond, -O-, -C(=O)-, and -C(=O)O-.

The disclosure (7) relates to the thermoplastic resin composition according to any one of the disclosures (1) to (6), wherein the resin (A) includes at least one selected from the group consisting of an ethylene-vinyl alcohol copolymer and polylactic acid.

The disclosure (8) relates to the thermoplastic resin composition according to any one of the disclosures (1) to (7), wherein the resin (A) has a dispersed particle size of 5 to 100 μm.

The disclosure (9) relates to the thermoplastic resin composition according to any one of the disclosures (1) to (8), wherein the thermoplastic resin (B) includes a polyolefin resin.

The disclosure (10) relates to the thermoplastic resin composition according to any one of the disclosures (1) to (9), wherein the thermoplastic resin (B) includes metallocene-catalyzed linear low-density polyethylene.

The disclosure (11) relates to the thermoplastic resin composition according to any one of the disclosures (1) to (10), wherein the thermoplastic resin composition includes a masterbatch, and the resin (A) is contained in an amount of 8 to 50% by mass.

The disclosure (12) relates to the thermoplastic resin composition according to any one of the disclosures (1) to (10), wherein the thermoplastic resin composition includes: a masterbatch containing the resin (A) and the thermoplastic resin (B); and a thermoplastic resin (C), and the resin (A) is contained in an amount of 0.1 to 1.0% by mass.

The disclosure (13) relates to the thermoplastic resin composition according to the disclosure (12), wherein the thermoplastic resin (C) includes metallocene-catalyzed linear low-density polyethylene.

The disclosure (14) relates to a molded article containing the thermoplastic resin composition according to any one of the disclosures (1) to (13).

The disclosure (15) relates to the molded article according to the disclosure (14), wherein the molded article is in a form of a tube, a film, or a sheet.

The disclosure (16) relates to a method for producing the thermoplastic resin composition according to any one of the disclosures (1) to (13), including a mixing step of mixing the resin (A) and the thermoplastic resin (B) using a twin-screw extruder.

The disclosure (17) relates to the method for producing the thermoplastic resin composition according to the disclosure (16), wherein the twin-screw extruder includes a screw in which multiple screw elements, including two or more kneading disk elements, are mounted on a shaft and a barrel housing two of the screws, and

a kneading area ratio, a value obtained by dividing a total length of the kneading disk elements by a total length of the screw, is 0.01 or more.

The disclosure (18) relates to a method for evaluating a thermoplastic resin composition, including: a melting step of melting a thermoplastic resin composition containing a resin (A) and a thermoplastic resin (B) by heating at a temperature increase rate of 5°C to 15°C/min to 180°C to 200°C, followed by standing for 3 to 10 minutes; and

an observing step of observing the thermoplastic resin composition after melting under a polarizing microscope to evaluate a dispersed particle size of the resin (A).

- Advantageous Effects of Invention

The disclosure can provide a thermoplastic resin composition having good processibility, a molded article, and a method for producing a thermoplastic resin composition. The disclosure also can provide a method for evaluating a thermoplastic resin composition, which enables highly accurate evaluation of the dispersed particle size of the resin.

BRIEF DESCRIPTION OF THE DRAWINGS

FIG. 1 is a schematic structural view showing a twin-screw extruder used in melt-kneading in EXAMPLES.

DESCRIPTION OF EMBODIMENTS

The disclosure is described in detail below.

<Thermoplastic resin composition>

A thermoplastic resin composition of the disclosure contains a resin (A) and a thermoplastic resin (B). The resin (A) contains a structural unit represented by the following formula 1 and has a dispersed particle size of 1 to 100 μm.

$$-X-(CR^1R^2)_n-Y-(CR^3R^4)_m-Z- \qquad \text{(formula 1)}$$

In the formula 1, X is a single bond or a divalent group optionally containing a functional group;

Y and Z are each independently a group constituted by at least one selected from the group consisting of a single bond, -O-, -C(=O)-, -C(=O)O-, -OC(=O)O-, -C(=NR')-, - C(=NR')O-, -OC(=NR')O-, -S-, -S(=O)-, -S(=O)O-, -OS(=O)O-, $-S(=O)_2-$, $-S(=O)_2O-$, $-OS(=O)_2O-$, -P(=O)-, -P(=O)O-, - OP(=O)O-, $-P(=O)_2-$, $-P(=O)_2O-$, $-OP(=O)_2O-$, -NR'-, and - C(OR')R'-, wherein R's at respective occurrences are each independently a hydrogen atom or a C1-C10 hydrocarbon group;
$R^1$, $R^2$, $R^3$, and $R^4$ are each independently a hydrogen atom or a C1-C10 hydrocarbon group; and
n and m are each independently an integer of 0 to 10,
with at least one of X, Y, or Z being -C(=O)-, - C(=O)O-, -OC(=O)O-, or -C(OR')R'-.

With the thermoplastic resin composition of the disclosure, good processibility can be achieved. In particular, good extrusion processibility can be achieved even in long-run molding. The development of the thermoplastic resin composition of the disclosure is based on the finding that the dispersed particle size of the resin (A) strongly correlates with processibility and that maintaining the dispersed particle size of the resin (A) within a specific range leads to particularly good processibility.

In the formula 1, preferably, X is a divalent group constituted by at least one selected from the group consisting of $X^1$ and $X^2$,
wherein $X^1$ is a group constituted by at least one selected from the group consisting of -C(=O)-, -C(=NR')-, $-S(=O)_2-$, -NR'-, -CR'R'-, and -C(OR')R'-, wherein R's at respective occurrences are each independently a hydrogen atom or a C1-C10 hydrocarbon group; and
$X^2$ is a C1-C12 aromatic hydrocarbon group optionally containing a substituent.

In the formula 1, X is more preferably a divalent group containing at least one selected from the group consisting of -C(=O)-, -CR'R'-, and -C(OR')R'-, wherein R's at respective occurrences are each independently a hydrogen atom or a C1-C10 hydrocarbon group.

In the formula 1, R's at respective occurrences are preferably each independently a hydrogen atom or a C1-C7 hydrocarbon group, more preferably a hydrogen atom or a C1-C4 hydrocarbon group, still more preferably a hydrogen atom or a C1 hydrocarbon group.

In the formula 1, Y and Z are preferably each independently a group constituted by at least one selected from the group consisting of a single bond, -O-, -C(=O)-, - C(=O)O-, -C(=NR')-, -C(=NR')O-, -S-, $-S(=O)_2-$, $-S(=O)_2O-$, - NR'-, and -C(OR')R'-, wherein R's at respective occurrences are each independently a hydrogen atom or a C1-C10 hydrocarbon group.

In the formula 1, Y and Z are preferably each independently a group constituted by at least one selected from the group consisting of a single bond, -O-, -C(=O)-, and -C(=O)O-.

In the formula 1, $R^1$, $R^2$, $R^3$, and $R^4$ are preferably each independently a hydrogen atom or a C1-C7 hydrocarbon group, more preferably a hydrogen atom or a C1-C4 hydrocarbon group, still more preferably a hydrogen atom or a C1 hydrocarbon group.

In the formula 1, n and m are preferably each independently an integer of 0 to 8, more preferably an integer of 0 to 6, still more preferably an integer of 0 to 4, particularly preferably an integer of 0 to 2.

The resin (A) may contain a structural unit other than the structural unit represented by the formula 1. Examples of the structural unit other than the structural unit represented by the formula 1 include structural units derived from: glycol compounds such as ethylene glycol, propylene glycol, butanediol, heptanediol, hexanediol, octanediol, nonanediol, decanediol, 1,4-cyclohexanedimethanol, neopentyl glycol, glycerol, pentaerythritol, bisphenol A, polyethylene glycol,

polypropylene glycol, and polytetramethylene glycol; dicarboxylic acids such as oxalic acid, adipic acid, sebacic acid, azelaic acid, dodecanedioic acid, malonic acid, glutaric acid, cyclohexanedicarboxylic acid, terephthalic acid, isophthalic acid, phthalic acid, naphthalenedicarboxylic acid, bis(p-carboxyphenyl)methane, anthracenedicarboxylic acid, 4,4'-diphenyl ether dicarboxylic acid, sodium 5-sulfoisophthalate, and tetrabutylphosphonium 5-isophthalate; hydroxycarboxylic acids such as glycolic acid, hydroxypropionic acid, hydroxybutyric acid, hydroxyvaleric acid, hydroxycaproic acid, and hydroxybenzoic acid; and lactones such as caprolactone, valerolactone, propiolactone, undecalactone, and 1,5-oxepan-2-one.

The amount of the structural unit represented by the formula 1 in the resin (A) is preferably 0.1% by mass or more, more preferably 1% by mass or more, still more preferably 10% by mass or more, particularly preferably 20% by mass or more, while preferably 99.9% by mass or less, more preferably 99% by mass or less, still more preferably 95% by mass or less.

Examples of the resin (A) include polylactic acid (PLA), polybutylene succinate (PBS), an ethylene-vinyl alcohol copolymer (EVOH), and polybutylene succinate adipate (PBSA). The resin (A) preferably includes at least one selected from the group consisting of PLA, PBS, EVOH, and PBSA, more preferably at least one selected from the group consisting of PLA, PBS, and EVOH, still more preferably at least one selected from the group consisting of PLA and EVOH.

The ethylene-vinyl alcohol copolymer has an ethylene content of preferably 10 mol% or more, more preferably 20 mol% or more, still more preferably 30 mol% or more, while preferably 60 mol% or less, more preferably 50 mol% or less, still more preferably 40 mol% or less. The ethylene-vinyl alcohol copolymer having an ethylene content within this range can lead to a better effect of improving the processibility.

The ethylene content herein is determined by nuclear magnetic resonance (NMR) analysis.

The ethylene-vinyl alcohol copolymer is preferably one obtained by saponifying an ethylene-vinyl ester copolymer, and is particularly preferably one obtained by saponifying an ethylene-vinyl acetate copolymer.

The ethylene-vinyl alcohol copolymer has a degree of saponification of preferably 80 to 100 mol%.

Copolymerization of ethylene and vinyl acetate may involve use of a different fatty acid vinyl ester (e.g., vinyl propionate or vinyl pivalate). The ethylene-vinyl alcohol copolymer may contain 0.0002 to 0.2 mol% of a vinylsilane compound as a copolymerized component. Examples of the vinylsilane compound include vinyltrimethoxysilane, vinyltriethoxysilane, vinyltri($\beta$-methoxy-ethoxy)silane, and $\gamma$-methacryloxypropylmethoxysilane. Preferably used among these are vinyltrimethoxysilane and vinyltriethoxysilane.

Copolymerization of ethylene and vinyl acetate may involve the presence of a small amount of a monomer different from the aforementioned fatty acid vinyl ester or vinylsilane compound, such as any of $\alpha$-olefins such as propylene, isobutylene, $\alpha$-octene, and $\alpha$-dodecene; unsaturated acids such as acrylic acid, methacrylic acid, crotonic acid, maleic acid, and itaconic acid, and anhydrides, salts, and mono- or dialkyl esters thereof; nitriles such as acrylonitrile and methacrylonitrile; amides such as acrylamide and methacrylamide; olefin sulfonic acids such as ethylene sulfonic acid, allyl sulfonic acid, and methallyl sulfonic acid and salts thereof; alkyl vinyl ethers, vinyl ketones, N-vinylpyrrolidone, vinyl chloride, and vinylidene chloride.

The melting point of the resin (A) is preferably 65°C or higher, more preferably 70°C or higher, still more preferably 75°C or higher, further preferably 80°C or higher, while preferably 190°C or lower, more preferably 185°C or lower, still more preferably 180°C or lower. The resin (A) having a melting point within this range can lead to a better effect of improving the processibility.

The melting point herein is the temperature corresponding to the maximum value on a heat-of-fusion curve at a temperature increase rate of 10°C/min using a differential scanning calorimeter (DSC).

The resin (A) has a melt flow rate (MFR) of preferably 0.001 g/10 min or higher, more preferably 0.01 g/10 min or higher, still more preferably 0.05 g/10 min or higher, further preferably 0.1 g/10 min or higher, particularly preferably 0.5 g/10 min or higher, while preferably 500 g/10 min or lower, more preferably 300 g/10 min or lower, still more preferably 150 g/10 min or lower, further preferably 40 g/10 min or lower, further preferably 20 g/10 min or lower, particularly preferably 10 g/10 min or lower. The resin (A) having a MFR within this range can lead to a better effect of improving the processibility.

The MFR herein is determined under conditions of 190°C and a 2.16 kgf load in conformity with ASTM D1238.

From the viewpoint of improved processibility, the weight average molecular weight of the resin (A) is preferably 80000 or more, more preferably 100000 or more. From the same viewpoint, the weight average molecular weight of the resin (A) is preferably 400000 or less, more preferably 350000 or less. The weight average molecular weight of the resin (A) can be determined by gel permeation chromatography (GPC). The analysis is performed using chloroform as a solvent with a high-temperature SEC column (GMHHR-H series) available from Tosoh Corporation at a flow rate of 1.0 mL/min and a column temperature of 40°C. A differential refractive index detector (RI) is used as a detector, with polystyrene of known molecular weight as a reference.

Examples of the thermoplastic resin (B) include a polyolefin polymer (e.g., polyethylene (PE), polypropylene (PP), and an ethylene-propylene copolymer), polystyrene (PS), acrylonitrile styrene (AS) resin (AS), acrylonitrile butadiene styrene (ABS) resin (ABS), methacrylic resin (PMMA), polymethyl pentene (PMP), polybutadiene resin (BDR), polybutene-1 (PB-1), polyvinyl alcohol (PVA), polyacrylonitrile (PAN), polystyrene methacrylate (MS), an ethylene-vinyl acetate copolymer (EVA), an ethylene-vinyl alcohol copolymer, and polyvinyl chloride (PVC). Each of these may be used alone or two or more of these may be used in combination. To achieve a better effect of improving the processibility, a polyolefin polymer (polyolefin resin) is preferred.

An example of the polyolefin polymer is a homopolymer (e.g., a homopolymer of a C2-C10 α-olefin, preferably a C2-C6 α-olefin). Specific examples of the homopolymer include homo-polyethylene and homo-polypropylene (hPP). In the case of homo-polyethylene, for example, this polymer can be produced by free-radical polymerization in a high-pressure process and is typically known as a highly branched ethylene homopolymer, which is often referred to as LDPE (low-density polyethylene), having a density of lower than 0.945 g/cm$^3$, often 0.935 g/cm$^3$ or lower, e.g., within a range from 0.900, 0.905, or 0.910 g/cm$^3$ to 0.920, 0.925, 0.927, 0.930, 0.935, or 0.945 g/cm$^3$. Unless otherwise described herein, the densities of all polymers are determined in conformity with ASTM D1505. A sample is formed through the step C of ASTM D4703-10a and left under the conditions in ASTM D618-08 (23 ± 2°C and relative humidity 50 ± 10%) for 40 hours before testing.

In another example, an ethylene monomer can be polymerized in a phase of a known gas, slurry, and/or solution (using, e.g., a catalyst such as chromium catalyst or a single site catalyst such as Ziegler-Natta and/or metallocene catalyst), each of which is well known in the polymerization technique and is therefore not elaborated herein. In the case of producing a more highly linear ethylene homopolymer (e.g., using gas-phase or slurry-phase polymerization with any of the above catalysts), this is referred to as HDPE (high-density polyethylene), which typically has a density of 0.945 g/cm$^3$ or higher, e.g., within a range from 0.945 to 0.970 g/cm$^3$.

Examples of the polymer also include copolymers of two or more C2-C40 α-olefins, e.g., C2-C20 α-olefins, such as ethylene-α-olefin copolymers and propylene-α-olefin copolymers (e.g., a propylene-ethylene copolymer and a propylene-ethylene-diene terpolymer (also known as EPDM and PEDM)). Specific examples intended herein include an ethylene copolymer and one or more C3-C20 α-olefin comonomers such as a C4-C12 α-olefin comonomer (1-butene, 1-hexene, 1-octene, or a mixture of any two or more of these is preferred in a variety of embodiments). The ethylene copolymer (e.g., a copolymer of ethylene and one or more C3-C20 α-olefins) may contain an ethylene-derived unit in an amount of at least 90, 94, 95, or 96% by weight (e.g., within a range from a lower value of 80, 85, 90, 80, 85, 93, 93, 95, 96, or 97 wt% to a higher value of 94, 95, 95.5, 91, 92, 97, 94, 97.5, or 98 wt%; at least 96 wt% or 96.5 wt%), and may contain the unit in an amount within the range from any of the lower values to any of the higher values based on the total amount of the ethylene-derived unit and a comonomer-derived unit. For example, the ethylene copolymer may contain 94 or 95% by mass to 97 or 98% by mass of an ethylene-derived unit based on the total amount of the ethylene-derived unit and a comonomer-derived unit. The balance of the copolymer (based on the ethylene-derived unit and the comonomer-derived unit) consists of the comonomer-derived unit. For example, a comonomer unit (e.g., a unit derived from a C2-C20 α-olefin such as a unit derived from butene, hexene, and/or octene) may be present in an amount within a range from a lower value of 2, 2.5, 3, 3.5, 4, 4.5, 5, or 6% by mass to a higher value of 3, 4, 5, 6, 7, 8, 9, 10, 15, or 20% by mass, and the range thereof is from any of the above lower values to any intended value among the above higher values (provided that the higher value is greater than the lower value).

Some appropriate comonomers have already been known for ethylene-based, propylene-based, or other α-olefin-based copolymers; in various embodiments, other α-olefin comonomers are intended. For example, the α-olefin comonomer may be linear or branched, and two or more comonomers may be used as appropriate. Examples of appropriate comonomers include C3-C20 linear α-olefins (e.g., butene, hexene, and octene, as described above) and α-olefins having one or more C1-C3 alkyl branches or aryl groups. Examples include: propylene; 3-methyl-1-butene; 3,3-dimethyl-1-butene; 1-pentene; 1-pentene having one or more methyl, ethyl, or propyl substituents; 1-hexene having one or more methyl, ethyl, or propyl substituents; 1-heptene having one or more methyl, ethyl, or propyl substituents; 1-octene having one or more methyl, ethyl, or propyl substituents; 1-nonene having one or more methyl, ethyl, or propyl substituents; ethyl-, methyl- or dimethyl-substituted 1-decene; 1-dodecene; and styrene. The above list of comonomers shows mere examples and is not intended to set any limitation. In some embodiments, the comonomer includes propylene, 1-butene, 1-pentene, 4-methyl-1-pentene, 1-hexene, 1-octene, and styrene.

In a specific embodiment, the polymer may include an ethylene copolymer or may be an ethylene copolymer (according to the above description). The ethylene copolymer can be produced by gas, slurry, or solution phase polymerization. Some particularly preferred ethylene copolymers can be produced by gas or slurry phase polymerization. Specific examples include linear low-density polyethylene (LLDPE) and a copolymer of ethylene and one or more α-olefins, which are polymerized in the presence of one or more single site catalysts, e.g., one or more Ziegler-Natta catalysts, one or more metallocene catalysts, or combination of any of these. This LLDPE may have a density within a range from a lower concentration of 0.900, 0.905, 0.907, or 0.910 g/cm$^3$ to a higher concentration of 0.920, 0.925, 0.930, 0.935, 0.940, or 0.945 g/cm$^3$. LLDPE is distinguishable from the aforementioned LDPE in some respects, many of which are well-known in the technical field, including the degree of branching (in many cases, there are few branches, if present) in the polymer produced. It should be noted that LLDPE has substantially a small number of long chain branches. In a specific embodiment, the polymer of the polymer composition is or includes a metallocene-catalyzed LLDPE (mLLDPE). In other embodiments, the polymer of the polymer composition is or includes a Ziegler-Natta-catalyzed LLDPE (or ZN-LLDPE).

In some embodiments, the density of the polymer falls within a range from 0.905 to 0.945 g/cm$^3$, e.g., from a lower value of 0.905, 0.907, 0.908, 0.910, 0.911, 0.912, 0.913, 0.914, or 0.915 g/cm$^3$ to a higher value of 0.916, 0.917, 0.918, 0.919, 0.920, 0.924, 0.926, 0.930, 0.935, 0.940, or 0.945 g/cm$^3$, and the range thereof is from any of the above lower values to any of the above higher values intended herein (e.g., from 0.910 to 0.925 or 0.935 g/cm$^3$, e.g., from 0.912 to 0.925 or from 0.915 to 0.918 g/cm$^3$). In other embodiments, the polymer may be a higher-density one (e.g., HDPE) having a density within a range from 0.945 g/cm$^3$ to 0.970 g/cm$^3$.

The rheological characteristics of a polymer can have influence on the processing aid composition for forming a molded article. In common cases, a PPA composition is preferably used in a polymer having a melt index (MI or I2 is measured at 190°C and a 2.16 kg load in conformity with ASTM D1238) of 1.5 g/2.0 min or lower, preferably 2.5 g/3.0 min or lower, such as within a range from 0.1, 0.2, or 0.5 g/10 min to 1.0, 1.2, 5.0, 10, 2.5, 10, 4.0, or 5.0 g/10 min. In some embodiments, the polymer may commonly have a melt index ratio (MIR) (MIR herein is defined as the ratio of the high-load melt index (HLMI) (determined at 190°C and a 21.6 kg load per ASTM D1238) to the melt index or as HLMI/MI) within a range from 10, 12, or 15 to 19, 20, 21, 22, 25, 27, 30, 35, 40, 45, 50, 60, 65, 70, 75, 80, 85, 90, 95, or 100. If necessary, this polymer may have a MI of lower than 1.5 g/10 min, such as 1.0 g/10 min or lower (e.g., from 0.1, 0.2, or 0.5 g/10 min to 1.0; or any of 1.1, 1.2, 1.3, or 1.4, or lower than 1.5 g/10 min).

The LLDPE preferably includes at least one selected from the group consisting of a Ziegler-Natta-catalyzed LLDPE and a metallocene-catalyzed LLDPE. Particularly preferred is a metallocene-catalyzed LLDPE.

The thermoplastic resin (B) may have crystallinity or may have no crystallinity. The thermoplastic resin (B) having crystallinity has a melting point of preferably 80°C to 300°C, more preferably 100°C to 200°C. The thermoplastic resin (B) having no crystallinity preferably has a processing temperature substantially equal to that of a thermoplastic resin (B) whose melting point range is indicated by its crystallinity.

In the thermoplastic resin composition of the disclosure, the resin (A) is dispersed in the thermoplastic resin (B). In other words, the thermoplastic resin composition of the disclosure forms a sea-island structure in which the thermoplastic resin (B) forms a sea phase and the resin (A) forms an island phase.

In the thermoplastic resin composition of the disclosure, the dispersed particle size of the resin (A) is 1 to 100 μm. The dispersed particle size within this range leads to good processibility. The lower limit thereof is preferably 5 μm or greater and the upper limit thereof is preferably 80 μm or smaller, more preferably 50 μm or smaller, still more preferably 40 μm or smaller, particularly preferably 30 μm or smaller.

When the dispersed particle size of the resin (A) is 10 μm or greater, the melt fracture disappearance time is shortened. In terms of the melt fracture disappearance time, the dispersed particle size of the resin (A) is preferably 10 to 100 μm, more preferably 15 to 50 μm, still more preferably 30 to 50 μm.

When the dispersed particle size of the resin (A) is 50 μm or smaller, the amount of die build-up (DBU) is reduced. In order to prevent or suppress die build-up, the dispersed particle size of the resin (A) is preferably 1 to 50 μm, more preferably 5 to 30 μm.

The dispersed particle size of the resin (A) can be measured by the following technique.

(1) The thermoplastic resin composition in which the resin (A) is dispersed in the thermoplastic resin (B) is cut in a direction perpendicular to the extrusion direction using a microtome, whereby 10 pieces of samples with a thickness of about 30 μm are obtained. The resulting samples are each heated at a temperature increase rate of 10°C/min to 190°C and then allowed to stand for 10 minutes. Thus, the samples are melted.

(2) The melted thermoplastic resin composition samples are observed using a polarizing microscope (Nikon ECLIPSE LV100N POL (camera: Nikon DS-Fi2), magnification: 200X). Images are taken and analyzed using analysis software (NIS-Elements D) to measure the particle size. One image is taken for each sample, and 10 images in total are taken. The average of the particle sizes of the dispersed particles in the images is used as the dispersed particle size. When the particles are not in the circular form, the major axis is used as the particle size.

The dispersed particle size of the resin (A) can be adjusted by, for example, the conditions for mixing. Specifically, the dispersed particle size becomes smaller under the conditions including a stronger shear force to the target and the dispersed particle size becomes greater under the conditions including a smaller shear force to the target. The dispersed particle size of the resin (A) is adjusted to a preferred range from the standpoint of improvement of the processibility, for example, by the method for producing a thermoplastic resin composition of the disclosure described later.

The thermoplastic resin composition of the disclosure may be a masterbatch or a composition mixed or not mixed with a masterbatch. For better processibility, the thermoplastic resin composition of the disclosure is preferably a masterbatch or a composition mixed with a masterbatch.

When the thermoplastic resin composition of the disclosure is a masterbatch, the thermoplastic resin composition of the disclosure is particularly useful as a processing aid for thermoplastic resins (particularly, polyolefin resins).

When the thermoplastic resin composition of the disclosure is a masterbatch, the masterbatch has a melt flow

rate (MFR) of preferably 0.1 g/10 min or higher, more preferably 0.5 g/10 min or higher, still more preferably 1 g/10 min or higher, further preferably 1.5 g/10 min or higher, while preferably 100 g/10 min or lower, more preferably 50 g/10 min or lower, still more preferably 30 g/10 min or lower, further preferably 20 g/10 min or lower, particularly preferably 10 g/10 min or lower. The masterbatch having a melt flow rate within this range can lead to a better effect of improving the processibility.

When the thermoplastic resin composition of the disclosure is a masterbatch, the amount of the resin (A) is preferably 1% by mass or more, more preferably 3% by mass or more, still more preferably 5% by mass or more, particularly preferably 8% by mass or more, while preferably 80% by mass or less, more preferably 50% by mass or less, still more preferably 40% by mass or less, particularly preferably 30% by mass or less.

When the thermoplastic resin composition of the disclosure is a masterbatch, the mass ratio of the thermoplastic resin (B) to the resin (A) (thermoplastic resin (B):resin (A)) is preferably 99:1 to 1:99. The mass ratio is more preferably 97:3 to 50:50, still more preferably 95:5 to 60:40, particularly preferably 92:8 to 70:30.

When the thermoplastic resin composition of the disclosure is a masterbatch, the total amount of the thermoplastic resin (B) and the resin (A) is preferably 80% by mass or more, more preferably 90% by mass or more, still more preferably 95% by mass or more, particularly preferably 100% by mass.

When the thermoplastic resin composition of the disclosure is a composition mixed with a masterbatch or a composition not mixed with a masterbatch, the amount of the resin (A) is preferably 0.001% by mass or more, more preferably 0.08% by mass or more, still more preferably 0.1% by mass or more, while preferably 5% by mass or less, more preferably 3% by mass or less, still more preferably 2% by mass or less, particularly preferably 1.0% by mass or less.

When the thermoplastic resin composition of the disclosure is a composition mixed with a masterbatch, the composition is preferably a thermoplastic resin composition containing a masterbatch containing the resin (A) and the thermoplastic resin (B) and a thermoplastic resin (C). This leads to good processibility. In particular, good extrusion processibility can be achieved even in long-run molding. Moreover, the resulting extruded product has excellent storage stability.

The thermoplastic resin (C) may be the same as the thermoplastic resin (B), and a preferred form thereof is also the same as that of the thermoplastic resin (B). In the case where the thermoplastic resin composition of the disclosure is a composition mixed with a masterbatch, the thermoplastic resin (B) and the thermoplastic resin (C) may be the same as or different from each other.

The thermoplastic resin composition of the disclosure is preferably substantially free from fluorine. The phrase "substantially free from fluorine" means that the processing aid has a fluorine content of 10 ppm or less (preferably 1 ppm or less, more preferably 0.1 ppm or less). The processing aid of the disclosure is particularly preferably free from fluorine (has a fluorine content of 0% by mass).

The water content of the processing aid of the disclosure is preferably 1.0% by mass or less, more preferably 0.5% by mass or less, still more preferably 0.2% by mass or less, most preferably 0.1% by mass or less. The lower limit may be, but is not limited to, 0% by mass.

The water content herein is determined by the following method.

The mass of the thermoplastic resin composition of the disclosure is weighed before and after heating at 130°C for 24 hours, and the water content is calculated by the following equation. Three samples are taken, and this calculation is performed for each sample and the values are averaged.

Water content (% by mass) = [(Mass (g) of thermoplastic resin composition before heating) - (Mass (g) of thermoplastic resin composition after heating)]/(Mass (g) of thermoplastic resin composition before heating) × 100

The thermoplastic resin composition having a water content within the above range can be obtained by any method. For example, a thermoplastic resin composition may be produced using materials with low water content under dry conditions, or a thermoplastic resin composition may be produced using normal materials under normal conditions and water may be removed therefrom by, for example, heat treatment.

The thermoplastic resin composition of the disclosure may contain a component different from the resin (A), the thermoplastic resin (B), and the thermoplastic resin (C). Examples of the different component include a synergist including at least one selected from the group consisting of a polyol having a melting point of 80°C or lower, polycaprolactone, silicone, and a polyamide-polyether block copolymer.

The melting point of the polyol is at least 80°C or lower, preferably 75°C or lower, more preferably 70°C or lower, still more preferably 68°C or lower, while preferably 10°C or higher, more preferably 20°C or higher, still more preferably 25°C or higher. The polyol having a melting point within this range can lead to a better effect of improving the processibility.

The polyol can be represented by $A[(OR^{11})_xOR^{12}]_y$, wherein A is commonly alkylene having one or more ether bonds; y is 2 or 3, $(OR^{11})_x$ is a poly(oxyalkylene) chain having multiple (x) oxyalkylene groups $OR^{11}$; $R^{11}$s are each independently C2-C5 alkylene or, in some embodiments, C2-C3 alkylene; $R^{12}$ is hydrogen, alkyl, aryl, arylalkenyl, alkylarylenyl, -C(O)-alkyl, -C(O)-aryl, -C(O)-arylalkenyl, or -C(O)-alkylarylenyl, where -C(O)- bonds to O of $OR^{12}$; and x is

10 to 230000.

The polyol may be, for example, a homopolymer such as poly (oxypropylene) in which $R^{11}$ is $-CH_2CH_{2-}$ or poly (oxyethylene) in which $R^{11}$ is $-C_3H_6-$.

The polyol may be a chain of randomly distributed oxyalkylene groups (e.g., $-OC_2H_4-$ and $-OC_3H_6-$ units which are copolymers) or a chain including alternating blocks of repeated oxyalkylene groups (e.g., a polymer including a $(-OC_2H_4-)_{a1}$ block and a $(-OC_3H_6-)_{b1}$ block, where a1 + b1 is 10 to 230000).

In some embodiments, A in the polyol is ethylene, $-CH_2-CH(-)-CH_2-$ (derived from glycerol), $CH_3CH_2C(CH_2-)_3$ (derived from 1,1,1-trimethylolpropane), poly(oxypropylene), $-CH_2CH_2-O-CH_2CH_2-$, or $-CH_2CH_2-O-CH_2CH_2-O-CH_2CH_2-$; and $R^{12}$ is hydrogen, methyl, butyl, phenyl, benzyl, acetyl, benzoyl, or stearyl.

The polyol may be polyester prepared from a dicarboxylic acid and a poly(oxyalkylene) polymer represented by A $[(OR^{11})_{x2}OR^{12}]_{y2}$, wherein A, $R^{11}$, and x2 are as defined above; $R^{12}$ is hydrogen; and y2 is 2.

One of the polyols may be used alone or two or more thereof may be used in combination. To achieve an excellent effect of improving the processibility, polyethylene glycol or polyethylene oxide is preferred, and polyethylene glycol is particularly preferred.

The polyethylene glycol has a number average molecular weight (Mn) of preferably 1000 or higher, more preferably 3000 or higher, still more preferably 5000 or higher, while preferably 50000 or lower, more preferably 45000 or lower, still more preferably 40000 or lower. The polyethylene glycol having a number average molecular weight within this range can lead to a better effect of improving the processibility.

The number average molecular weight herein is determined by calculation from the hydroxyl value determined in conformity with JIS K0070.

The polyethylene oxide has a viscosity average molecular weight (Mv) of preferably 100000 or higher, more preferably 120000 or higher, still more preferably 140000 or higher, while preferably 10000000 or lower, more preferably 1600000 or lower, still more preferably 500000 or lower. The polyethylene oxide having a viscosity average molecular weight within this range can lead to a better effect of improving the processibility.

The viscosity average molecular weight herein is calculated as follows.

An Ostwald viscometer is used to determine at 35°C the specific viscosities ηsp of aqueous solutions containing a polymer at various concentrations c (g/dl) in pure water; each specific viscosity is divided by the corresponding polymer concentration to provide the reduced viscosity; based on the relationship between the resulting reduced viscosity (ηsp/c) and the polymer concentration c, with the polymer concentration c being extrapolated to 0, the [η] value is calculated. The [η] value is substituted into the following formula, whereby the viscosity average molecular weight M is calculated.

$$\text{Formula: } [\eta] = 6.4 \times 10^{-5} M^{0.82}$$

The polycaprolactone may be a homopolymer of ε-caprolactone or may be a modified polycaprolactone. Examples of the modified polycaprolactone include those modified with, for example, 1,4-butanediol combined during ring-opening polymerization of ε-caprolactone and those modified with, for example, an ether or ester group at an end of a polymer.

The polycaprolactone has a weight average molecular weight (Mw) of preferably 2000 or higher, more preferably 10000 or higher, still more preferably 25000 or higher, while preferably 100000 or lower, more preferably 95000 or lower, still more preferably 90000 or lower. The polycaprolactone having a weight average molecular weight within this range can lead to a better effect of improving the processibility.

The weight average molecular weight herein is determined in polystyrene equivalent by gel permeation chromatography (GPC).

The polycaprolactone has a melting point of preferably 80°C or lower, more preferably 75°C or lower, still more preferably 70°C or lower, particularly preferably 68°C or lower, while preferably 10°C or higher, more preferably 20°C or higher, still more preferably 45°C or higher. The polycaprolactone having a melting point within this range can lead to a better effect of improving the processibility.

The silicone may be basically any organic silicon compound known to those skilled in the art with the term silicone polymer. An appropriate definition of the silicone is found in Winnacker/Kuchler: "Chemische Technik" [Chemical Technology], R. Dittmeyer, W. Keim, G. Kreysa, A. Oberholz (editor), Vol. 5: "Organische Zwischenverbindungen, Polymere" [Organic Intermediates, Polymers], Chapter: "Silicones", Wiley-VCH, Weinheim, 2005.

The silicone may be a substituted or unsubstituted linear oligo- or polydiorganosiloxane, a branched silicone polymer, silicone resin, or a crosslinked silicone polymer. A mixture of various silicone polymers may also be used, of course. As described above, a silicone-containing copolymer may be used, such as a polyether functional silicone, a silicone containing a urea or urethane unit, or a silicone block copolymer with an organic polymer. To achieve better additivity, particularly preferred is to use a high molecular weight polydiorganosiloxane that can contain a non-silicone component, such as filler, e.g., fine silicic acid particulate, chalk, talc, or a sheet-shaped silicic acid salt.

Preferably, the silicone polymer corresponds to the formula A $[R^{13}{}_3SiO_{1/2}]_{a2}[SiR^{13}{}_2O_{2/2}]_{b2}[R^{13}SiO_{3/2}]_{c2}[SiO_{4/2}]_{d2}$, wherein $R^{13}$ is hydrogen, -OH, or a C1-C18 substituted or unsubstituted hydrocarbon residue; and a2, b2, c2, and d2 each mean 0 or an integer, with a2 + b2 + c2 + d2 being an integer of 5 to 15000.

Examples of the C1-C18 hydrocarbon residue $R^{13}$ include alkyl residues, such as methyl, ethyl, n-propyl, isopropyl, n-butyl, 2-butyl, isobutyl, tert-butyl, n-pentyl, isopentyl, neopentyl, a tert-pentyl residue, hexyl residues, e.g., a n-hexyl residue, heptyl residues, e.g., a n-heptyl residue, octyl residues, e.g., a n-octyl residue and an isooctyl residue, a 2,2,4-trimethylpentyl residue, nonyl residues, e.g., a n-nonyl residue, decyl residues, e.g., a n-decyl residue, cycloalkyl residues, e.g., a cyclopentyl residue, a cyclohexyl residue, a 4-ethylcyclohexyl residue, and a cycloheptyl residue, a norbornyl residue, and a methylcyclohexyl residue. Preferred among the alkyl residues are C1-C6 residues, such as methyl and ethyl residues, particularly a methyl residue.

Examples of $R^{13}$ also include C1-C18 unsaturated hydrocarbon residues such as alkenyl residues, e.g., a vinyl residue, a 2-propen-2-yl residue, an allyl residue, a 3-buten-1-yl residue, a 5-hexen-1-yl residue, and a 10-undecen-1-yl residue, as well as cycloalkenyl residues (a 2-cyclohexenyl residue, a 3-cyclohexenyl residue, a cyclopentadienyl residue, and a 2-(cyclohex-3-en-1-yl)ethyl residue); aryl residues, e.g., a phenyl residue, a biphenylyl residue, and a naphthyl residue; alkaryl residues, e.g., o-, m-, or p-tolyl residue and phenethyl residues (a 2-phenylethyl residue and a 1-phenylethyl residue); as well as aralkyl residues, e.g., a benzyl residue. Preferred C1-C18 unsaturated hydrocarbon residues $R^{13}$ are a vinyl residue and a phenyl residue.

Examples of a substituted hydrocarbon residue as the residue $R^{13}$ include halogenated hydrocarbons such as a chloromethyl residue, a 3-chloropropyl residue, a 3-bromopropyl residue, a 3,3,3-trifluoropropyl residue, and a 5,5,5,4,4,3,3-heptafluoropentyl residue, as well as a chlorophenyl residue, a dichlorophenyl residue, and a trifluorotolyl residue.

The residue $R^{13}$ is preferably coupled with a silicone polymer represented by the formula A via a Si-C bond, and may be coupled with the silicone polymer via an oxygen atom -O-.

$R^{13}$ preferably contains 1 to 6 carbon atoms. Particularly preferred are an ethyl residue, a phenyl residue, a vinyl residue, and a methyl residue.

Preferably, the number represented by a2 + b2 + c2 + d2 means at least 10, more preferably at least 100, particularly preferably at least 1000 and at most 15000, more preferably at most 10000, particularly preferably at most 7000.

Preferably, the number represented by c2 + d2 means $< 0.1 \times$ (a2 + b2 + c2 + d2), particularly c2 + d2 $< 0.05 \times$ (a2 + b2 + c2 + d2).

Preferably at least 50%, more preferably at least 70%, particularly preferably at least 80% of all residues $R^{13}$ means a methyl residue.

In principle, every silicone polymer corresponding to the formula (A) may be used. Still, preferred is a silicone polymer having a dynamic viscosity of higher than 1000 mPa·s, which is measured using a plate-cone system (cone CP50-2) having an opening angle of 2° and a diameter of 50 mm with a rheometer "MCR 302" available from Anton Paar at a measurement temperature of 25.00°C $\pm$ 0.05°C and a shear rate of 1 sec-1, preferably in conformity with DIN EN ISO3219:1994 and DIN 53019.

Among these silicones, a silicone polymer having a very high molecular weight may be used, such as UHMW polysiloxane (ultra-high molecular weight; described in K. J. Ryan, et al., Journal of Vinyl & Additive Technology, March 2000, Vol. 6, No. 1, pp. 7-19).

UHMW polysiloxane has a degree of polymerization within a range from > 1000 to about 14000, which corresponds to a number average molecular weight from 74 kg/mol to 1000 kg/mol.

Typical UHMW polysiloxane has a dynamic viscosity from 10 kPa·s to 50 kPa·s, preferably from 15 kPa·s to 30 kPa·s, which is determined using an airborne rotational rheometer preferably in conformity with DIN EN ISO3219:1994 and DIN 53019, where a plate-plate system (diameter 25 mm) having a measurement gap of 0.5 mm is used. The measurement temperature is 25.00°C +/- 0.1°C. The shear rate gradient is 0.1 $sec^{-1.}$ The viscosity described refers to an arithmetic mean of three distinct, independently measured values.

Owing to its inexpensiveness and effectiveness, particularly preferred among the UHMW polysiloxanes is a high molecular weight polydimethylsiloxane having a dynamic viscosity from 1 kPa·s to 50 kPa·s, preferably from 10 to 40 kPa·s, particularly preferably from 15 to 30 kPa·s (preferably determined by the aforementioned method).

Examples of the UHMW polysiloxane include commercially available UHMW polysiloxanes such as MULTIBASE® MB50-001 and MULTIBASE® MB50-002 available from Dupont, GENIOPLAST® PELLET S, GENIOPLAST® PELLET P PlUS, GENIOPLAST® PE50S08, and GENIOPLAST® PP50S12 available from Wacker Asahikasei Silicone Co., Ltd., and mixtures of any of these, with MB50-002 and GENIOPLAST® PELLET S being preferred.

The silicone polymer is commercially available in the form of pellets or granules or masterbatch for ready-to-use, which may be mixed with thermoplastic granules before additional processing.

The polyamide-polyether block copolymer is a copolymer including a polyamide block and a polyether block in the polymer backbone. In the disclosure, such a block copolymer including a polyamide block and a polyether block can

also be referred to as a "polyamide/polyether block copolymer". This may also be abbreviated to "PEBA copolymer" or "PEBA".

In some embodiments of the disclosure, the PEBA copolymer can be represented by the following formula:

[Chem. 1]

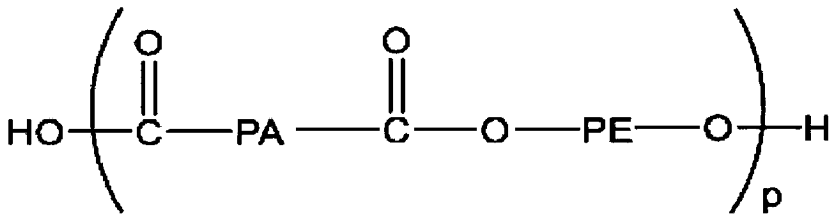

wherein PA represents a polyamide block, PE represents a polyether block, and p represents the length of the PEBA copolymer, which represents the total number of the polyamide and polyether blocks. In some embodiments of the disclosure, the PEBA copolymer can be represented by the following formula:

[Chem. 2]

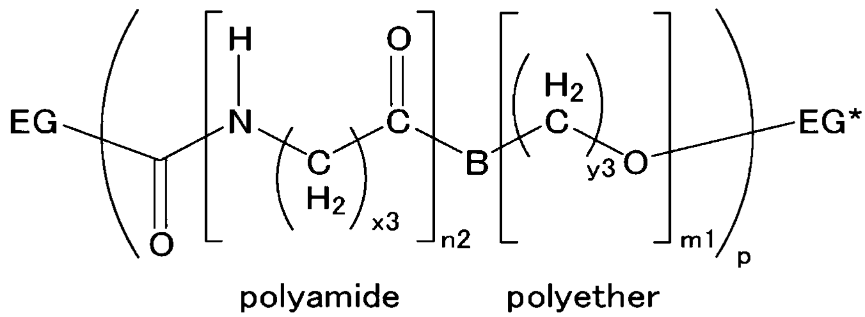

wherein EG represents a first unspecified end group; B represents an unspecified crosslinking group; and EG* represents a second unspecified end group, where EG, B, and EG* are determined by a synthesis method used for producing a PEBA copolymer, wherein n2 represents the length of the polyamide block; x3 represents the length of an amide component in the polyamide block; m1 represents the length of the poly(ether) block; y3 represents the length of an ether component in the poly(ether) block; and p represents the length of the PEBA copolymer, which represents the total number of the polyamide and polyether blocks.

In some embodiments of the disclosure, the PEBA copolymer can be represented by the following formula:

[Chem. 3]

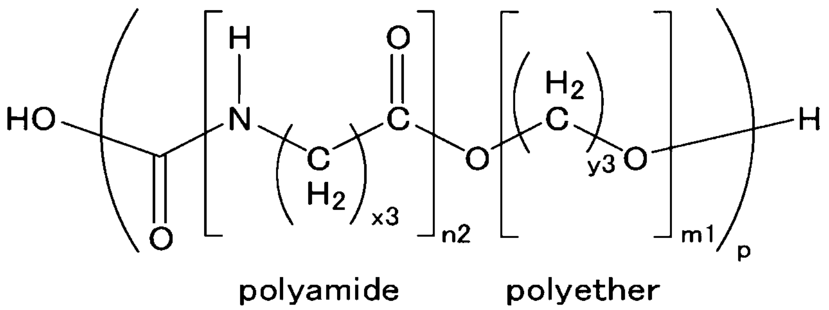

wherein n2 represents the length of the polyamide block; x3 represents the length of an amide component in the polyamide block; m1 represents the length of the poly(ether) block; y3 represents the length of an ether component in the poly(ether) block; and p represents the length of the PEBA copolymer, which represents the total number of the polyamide and polyether blocks.

The polyamide block in the PEBA copolymer is derived from polyamide-12 (PA-12), polyamide-11 (PA-11), polyamide-6 (PA-6), or polyamide-66 (PA-66).

The weight average molecular weight (e.g., Mw and Mn) of the PEBA copolymer can be determined, for example, by gel permeation chromatography using a narrow molecular weight polymer standard based on a known technique in the technical field (i.e., by size-exclusion chromatography).

In an embodiment, the number average molecular weight Mn of the polyamide block in the PEBA copolymer is about 100 to about 15000 g/mol, or about 300 to about 15000 g/mol, or about 600 to about 10000 g/mol, or about 600 to about 5000 g/mol.

The number average molecular weight Mn of the polyether block in the PEBA copolymer is about 100 to about 15000 g/mol, about 100 to about 10000 g/mol, about 100 to about 6000 g/mol, about 100 to about 3000 g/mol, about 200 to about 6000 g/mol, about 200 to about 3000 g/mol, about 250 to about 2000 g/mol, about 750 to about 3500 g/mol, or about 1000 to about 3000 g/mol.

The PEBA copolymer has a number average molecular weight Mn of 10000 to 500000 g/mol, including any subrange within these ranges and any number within these ranges. In an embodiment of the disclosure, for example, the number average molecular weight Mn of the PEBA copolymer is 10000 to 400000 g/mol, or 10000 to 300000 g/mol, 10000 to 250000 g/mol, or 15000 to 300000 g/mol, or 20000 to 300000 g/mol, or 15000 to 200000 g/mol, or 20000 to 200000 g/mol, or 30000 to 250000 g/mol, or about 25000 to about 75000 g/mol, or about 50000 to about 75000 g/mol, or about 100000 to about 150000 g/mol.

The number average molecular weight Mn of the PEBA copolymer is at least 10000 g/mol, at least 20000 g/mol, at least 25000 g/mol, exceeding 25000 g/mol, at least 30000 g/mol, exceeding 30000 g/mol, at least 35000 g/mol, exceeding 35000 g/mol, at least 50000 g/mol, or exceeding 50000 g/mol.

In an embodiment, the PEBA copolymer has a weight average molecular weight Mw of 25000 to 500000 g/mol, including any subrange within these ranges and any number within these ranges. In an embodiment of the disclosure, for example, the weight average molecular weight Mw of the PEBA copolymer is about 100000 to about 250000 g/mol, or about 100000 to about 150000 g/mol, or about 125000 to about 150000 g/mol.

The polyamide and polyether blocks in the PEBA copolymer may be randomly distributed.

The PEBA copolymer contains a polyamide block and a polyether block and the polyamide block may occupy at least 50% by mass of the copolymer. The PEBA copolymer contains a polyamide block and a polyether block and the polyether block may occupy at least 50% by mass of the copolymer. The PEBA copolymer contains a polyamide block and a polyether block and the ratio by mole of the polyamide block to the polyether block may fall within a range of 1:3 to 3:1, or 1:2 to 2:1, or 3:2 to 1:3, or 2:3 to 3:1, or about 1:1.

The PEBA copolymer containing a polyamide block and a polyether block can be prepared by reacting precursors of the polyamide and polyether blocks. For example, a lactam, a polyether diol, and a chain-limiting dioic acid may be reacted together in the presence of a small amount of water, thereby producing a length-variable PEBA copolymer containing a polyamide block and a polyether block statistically randomly distributed in the block copolymer chain.

The polyether block may be derived from poly(oxyethylene), poly(oxypropylene), or poly(tetramethylene ether) glycol, each of which in the natural state may be co-condensed with a polyamide block having a carboxylic acid chain end. A chain-limiting agent may be present during the polycondensation reaction to provide a PEBA copolymer containing a polyamide block and a polyether block randomly distributed in the block copolymer.

The polyether block is derived from poly(oxyethylene), poly(oxypropylene), or poly(tetramethylene ether)glycol, each of which are first aminated into polyether diamine and then co-condensed with a polyamide block having a carboxylic acid chain end. A chain-limiting agent may be present during the polycondensation reaction to provide a PEBA copolymer containing a polyamide block and a polyether block randomly distributed in the block copolymer.

The polyether block can be derived from poly(oxyethylene), which is also known as polyethylene glycol (PEG).

The polyether block can be derived from poly(oxypropylene), which is also known as polypropylene glycol (PPG).

The polyether block can be derived from poly(tetramethylene ether)glycol (PTMG), which is also known as polytetramethylene oxide (PTMEO) or polytetrahydrofuran (PTHF).

The PEBA copolymer contains i) a polyamide block selected from polyamide-12 (PA-12), polyamide-11 (PA-11), polyamide-6 (PA-6), or a mixture of any of these and ii) a polyether block selected from polyethylene glycol (PEG), polypropylene glycol (PPG), polytetrahydrofuran (PTHF), or a mixture of any of these.

The PEBA copolymer contains i) a polyamide block selected from polyamide-12 (PA-12), polyamide-11 (PA-11), polyamide-6 (PA-6), or a mixture of any of these and ii) a polyether block which is polyethylene glycol (PEG). In an embodiment of the disclosure, the PEBA copolymer contains i) a polyamide block which is polyamide-12 (PA-12) and ii) a polyether block which is polyethylene glycol (PEG).

The PEBA copolymer contains 10 to 20 polyamide blocks and 10 to 20 polyether blocks.

The PEBA copolymer contains a single species of the polyamide block and a single species of the polyether block.

The PEBA copolymer contains i) a polyamide block which is polyamide-12 (PA-12) and ii) a polyether block which is polyethylene glycol (PEG), wherein the polyamide-12 block occupies about 30% by mass to 70% by mass of the copolymer and the polyethylene glycol block occupies about 70% by mass to 30% by mass of the copolymer.

The PEBA copolymer contains i) a polyamide block which is polyamide-12 (PA-12) and ii) a polyether block which is polyethylene glycol (PEG), wherein the polyamide-12 block occupies about 40% by mass to 60% by mass of the copolymer and the polyethylene glycol block occupies about 60% by mass to 40% by mass of the copolymer.

The PEBA copolymer contains i) a polyamide block which is polyamide-12 (PA-12) and ii) a polyether block which is polyethylene glycol (PEG), wherein the polyamide-12 block represents about 45% by mass of the copolymer and the polyethylene glycol block represents about 55% by mass of the copolymer.

The PEBA copolymer contains i) 10 to 20 polyamide blocks each of which is polyamide-12 (PA-12) and ii) 10 to 20 polyether blocks each of which is polyethylene glycol (PEG).

The PEBA copolymer contains i) a polyamide block which is polyamide-12 (PA-12) and ii) a polyether block which is polyethylene glycol (PEG), and has a number average molecular weight Mn of about 25000 to about 75000 g/mol.

The PEBA copolymer contains i) a polyamide block which is polyamide-12 (PA-12) and ii) a polyether block which is polyethylene glycol (PEG), and has a number average molecular weight Mn of about 50000 to about 75000 g/mol. The PEBA copolymer contains i) a polyamide block which is polyamide-12 (PA-12) and ii) a polyether block which is polyethylene glycol (PEG), and has a number average molecular weight Mn of about 66100 g/mol.

The PEBA copolymer contains i) a polyamide block which is polyamide-12 (PA-12) and ii) a polyether block which is polyethylene glycol (PEG), and has a weight average molecular weight Mw of about 100000 to about 150000 g/mol.

The PEBA copolymer contains i) a polyamide block which is polyamide-12 (PA-12) and ii) a polyether block which is polyethylene glycol (PEG), and has a weight average molecular weight Mw of about 125000 to about 150000 g/mol.

The PEBA copolymer contains i) a polyamide block which is polyamide-12 (PA-12) and ii) a polyether block which is polyethylene glycol (PEG), and has a weight average molecular weight Mw of about 134000 g/mol.

In an embodiment of the disclosure, the PEBA copolymer contains i) a polyamide block which is polyamide-6 (PA-6) and ii) a polyether block which is polyethylene glycol (PEG).

The PEBA copolymer contains i) a polyamide block which is polyamide-6 (PA-6) and ii) a polyether block which is polyethylene glycol (PEG), wherein the polyamide-6 block represents about 30% by mass to 60% by mass of the copolymer and the polyethylene glycol block represents about 70% by mass to 40% by mass of the copolymer.

The PEBA copolymer contains i) a polyamide block which is polyamide-6 (PA-6) and ii) a polyether block which is polyethylene glycol (PEG), wherein the polyamide-6 block represents about 50% by mass to 35% by mass of the copolymer and the polyethylene glycol block represents about 50% by mass to 65% by mass of the copolymer.

The PEBA copolymer contains i) 10 to 20 polyamide blocks each of which is polyamide-6 (PA-6) and ii) 10 to 20 polyether blocks each of which is polyethylene glycol (PEG).

The PEBA copolymer contains i) a polyamide block which is polyamide-11 (PA-11) and ii) a polyether block which is polyethylene glycol (PEG). In an embodiment of the disclosure, the PEBA copolymer contains i) a polyamide block which is polyamide-12 (PA-12) and ii) a polyether block which is polytetrahydrofuran (PTHF).

The PEBA copolymer contains i) a polyamide block which is polyamide-12 (PA-12) and ii) a polyether block which is polytetrahydrofuran (PTHF), wherein the polyamide-12 block represents about 75% by mass to 10% by mass of the copolymer and the polytetrahydrofuran block represents about 25% by mass to 90% by mass of the copolymer.

The PEBA copolymer contains i) a polyamide block which is polyamide-12 (PA-12) and ii) a polyether block which is polytetrahydrofuran (PTHF), wherein the polyamide-12 block represents about 80% by mass to 60% by mass of the copolymer and the polytetrahydrofuran block represents about 20% by mass to 40% by weight of the copolymer.

The PEBA copolymer contains i) a polyamide block which is polyamide-12 (PA-12) and ii) a polyether block which is polytetrahydrofuran (PTHF), wherein the polyamide-12 block represents about 40% by mass to 60% by mass of the copolymer and the polytetrahydrofuran block represents about 60% by mass to 40% by mass of the copolymer.

The PEBA copolymer contains i) a polyamide block which is polyamide-12 (PA-12) and ii) a polyether block which is polytetrahydrofuran (PTHF), wherein the polyamide-12 block represents about 30% by mass to 10% by mass of the copolymer and the polytetrahydrofuran block represents about 70% by mass to 90% by mass of the copolymer.

The PEBA copolymer contains i) 10 to 20 polyamide blocks each of which is polyamide-12 (PA-12) and ii) 10 to 20 polyether blocks each of which is polytetrahydrofuran (PTHF).

The PEBA copolymer contains i) a polyamide block which is polyamide-12 (PA-12) and ii) a polyether block which is polytetrahydrofuran (PTHF), and has a number average molecular weight Mn of about 25000 to about 75000 g/mol.

The PEBA copolymer contains i) a polyamide block which is polyamide-12 (PA-12) and ii) a polyether block which is polytetrahydrofuran (PTHF), and has a number average molecular weight Mn of about 40000 to about 60000 g/mol. In an embodiment of the disclosure, the PEBA copolymer contains i) a polyamide block which is polyamide-12 (PA-12) and ii) a polyether block which is polytetrahydrofuran (PTHF), and has a number average molecular weight Mn of about 50000 g/mol.

The PEBA copolymer contains i) a polyamide block which is polyamide-6 (PA-6) and ii) a polyether block which is

polytetrahydrofuran (PTHF).

The PEBA copolymer contains i) a polyamide block which is polyamide-11 (PA-11) and ii) a polyether block which is polytetrahydrofuran (PTHF).

The PEBA copolymer is a commercially available elastomer and is marketed under the trade name of PEBAX®.

The PEBA copolymer is a commercially available elastomer selected from the group consisting of: PEBAX 2533 SA 01, PEBAX 2533 SA 01 MED, PEBAX 2533 SD 02, PEBAX 3533 SA 01, PEBAX 3533 SA 01 MED, PEBAX 3533 SP01, PEBAX 4011, PEBAX 4033 SA 01, PEBAX 4033 SA 01 MED, PEBAX 4033 SP01, PEBAX 4533 SA 01, PEBAX 4533 SA 01 MED, PEBAX 4533 SP01, PEBAX 5513 SA 01, PEBAX 5513 SP01, PEBAX 5533 SA 01, PEBAX 5533 SA 01 MED, PEBAX 5533 SN 70 BLACK, PEBAX 5533 SP01, PEBAX SA 01, PEBAX 6333 SA 01 MED, PEBAX SP01, PEBAX 6333 SP01, PEBAX 6333 SA 01, PEBAX, PEBAX 3533 SA 01, PEBAX 3533 SA 01 MED, PEBAX 3533 SP01, PEBAX 4011 SA 01, PEBAX 4033 SA 01 MED, PEBAX 4033 SP01, PEBAX 4033 SA 01 MED, PEBAX Clear 2533, PEBAX ES 2533 UV, PEBAX MH 2533, PEBAX MH2030, PEBAX MV 5513 SA 01, PEBAX MV 5513 SA 01 MED, PEBAX MV 5533 SP01, PEBAX MV 5533, PEBAX MV 5533 SP01, PEBAX RNEW® 30R51 SA 01, PEBAX RNEW 35R53 SP01, PEBAX RNEW 70R53 SP01, PEBAX RNEW 55R53 SP01, PEBAX RNEW 63R53 SP01, PEBAX RNEW 70R53 SP01, PEBAX RNEW 70R53 SP01, PEBAX, PEBAX RNEW 72R53 SP01, PEBAX RNEW 80R53 SP 02, and a mixture of any of these.

The PEBA copolymer is a commercially available elastomer and is marketed under the trade name of VESTAMID® or VESTAMID E.

The PEBA copolymer is a commercially available elastomer selected from the group consisting of VESTAMID D, VESTAMID DX, VESTAMID E, VESTAMID EX, VESTAMID Care, VESTAMID Care ML, VESTAMID Care ME, VESTAMID Care ME-B, VESTAMID L, VESTAMID LX, VESTAMID NRG, VESTAMID Terra, VESTAMID X, and a mixture of any of these. Preferred among these is Pebax MV 1072.

The PEBA copolymer may be used in the form of semisolid or viscous liquid, or in the form of powder, pellets, or granules.

Examples of the different component include an anti-agglomerating agent; an ultraviolet absorber; a flame retarder; a reinforcing material such as glass fiber or glass powder; a stabilizer such as mineral or flaky material; a lubricant such as silicone oil or molybdenum disulfide; a pigment such as titanium dioxide or red iron oxide; a conductive agent such as carbon black; an impact resistance improver such as rubber; an antioxidant such as a hindered phenol- or phosphorus-based antioxidant; a nucleating agent such as metal salt or acetal of sorbitol; and an antiblocking agent.

The thermoplastic resin composition of the disclosure can be obtained, for example, by a production method including a mixing step of mixing the resin (A) and the thermoplastic resin (B) using a twin-screw extruder. This can adjust the dispersed particle size of the resin (A) to a preferred range from the standpoint of improvement of the processibility. The disclosure also relates to a method for producing the aforementioned thermoplastic resin composition including the mixing step.

Use of a twin-screw extruder enables mixing (kneading) with application of shear force to materials, which enables easy adjustment of the dispersed particle size of the resin (A). Mixing (kneading) in this case is preferably melt-kneading.

In the case of melt-kneading, the components may be melted in the mixing step or may be melted before the mixing step.

Preferably, the twin-screw extruder includes a screw in which multiple screw elements, including two or more kneading disk elements, are mounted on a shaft and a barrel housing two of the screws, and the kneading area ratio, a value obtained by dividing the total length of the kneading disk elements by the total length of the screw, is 0.01 or more. The total lengths of the kneading disk elements and the screw are commonly measured in mm.

The twin-screw extruder has, for example, two screws, a barrel housing the two screws, a material feed port on the barrel, and a die at the downstream end of the barrel. The twin-screw extruder may further have a vacuum vent on the barrel, if needed.

The twin-screw extruder may be a co-rotating twin-screw extruder in which two screws through the cylinder of the barrel with an inverted V-shaped through-hole are rotated in the same direction or a counter-rotating twin-screw extruder in which such two screws are rotated in different directions.

The twin-screw extruder is preferably a co-rotating twin-screw extruder, because of its superior conveying capability, melting and kneading capability, and separation (dehydration) capability, as well as its ability to process materials continuously and enhance the efficiency of the resin composition production process.

The engagement of the two screws may be of a non-intermeshing type, a partially intermeshing type, or a fully intermeshing type.

As the screw, one capable of incorporating a later-described kneading area at any position on the screw is used. A screw in which multiple screw elements, including two or more kneading disk elements, are mounted on the shaft is used.

The screw elements have the same cross-sectional shape in the direction perpendicular to the axis. The screw elements have their specific functions depending on the number of strips, meaning the number of flights, and the helix angle at which the cross-sectional shape in the direction perpendicular to the shaft rotates around the shaft. Examples of

screw elements, classified by function, include rotary elements, kneading disk elements, and mixing elements.

The rotary element is a screw element that has a helix angle at which the element continuously rotates around the shaft and exhibits conveying capability.

The kneading disk element is a screw element that has no helix angle and is constituted by multiple plate-shaped disks.

The mixing element is a screw element that is a right-handed full-flight element with a notch or a screw element that is a left-handed full-flight element with a notch. The mixing element may or may not have self-cleanability.

As the screw in the twin-screw extruder, one constituted by a rotary element, a kneading disk element, and a mixing element is suitably used.

The kneading area ratio in the twin-screw extruder is preferably 0.02 or more, more preferably 0.05 or more, still more preferably 0.10 or more, particularly preferably 0.15 or more, most preferably 0.20 or more. The kneading area ratio is preferably 0.45 or less, more preferably 0.30 or less. When the kneading area ratio is equal to or more than the lower limit value, the resin (A) favorably disperses in the thermoplastic resin (B), and an excellent effect of improving the processibility is readily exhibited. When the kneading area ratio is equal to or less than the upper limit value, excessive shear heating of the thermoplastic resin A or B due to the screw and internal heating of the thermoplastic resin A or B due to deformation compression are suppressed, thereby preventing unnecessary decomposition of the thermoplastic resin A or B.

When the kneading area ratio is 0.10 or more, the resin (A) often has a dispersed particle size of 1 to 100 μm, and an excellent effect of improving the processibility tends to be exhibited.

When the kneading area ratio is 0.15 or more, the resin (A) often has a dispersed particle size of 1 to 50 μm, and a better effect of improving the processibility tends to be exhibited.

When the thermoplastic resin composition of the disclosure is a masterbatch and the resin (A) has a dispersed particle size of 50 μm or smaller, the value resulting from "kneading area ratio" × "concentration (% by mass) of resin (A) in masterbatch" is preferably 1 to 3 or 8 to 12 in order to shorten the melt fracture disappearance time.

When the thermoplastic resin composition of the disclosure is a masterbatch and the resin (A) has a dispersed particle size of 50 μm or smaller, the value resulting from "kneading area ratio" × "concentration (% by mass) of resin (A) in masterbatch" is preferably 3.1 to 8.5 in order to reduce the amount of die build up (DBU).

In the twin-screw extruder, the L/D is more preferably 20 or higher, more preferably 30 to 100 because the resin (A) and the thermoplastic resin (B) can be efficiently melted and kneaded. The "L/D" is a value obtained by dividing the total length L (mm) of the screw by the screw diameter D (mm).

The twin-screw extruder preferably has one or more melting zones in which two or more mixing elements and/or two or more kneading disk elements, among the screw elements, are arranged in succession. The twin-screw extruder with a melting zone enables melt-kneading of the resin (A), the thermoplastic resin (B), and the like. Two or more mixing elements and/or two or more kneading disk elements arranged in succession in the melting zone of the twin-screw extruder lengthen the residence time of the resin (A), the thermoplastic resin (B), and the like in the twin-screw extruder. When the resin (A), the thermoplastic resin (B), and the like pass through the melting zone, shear heat generated by the screw is applied to the resin (A), the thermoplastic resin (B), and the like so that the resin (A), the thermoplastic resin (B), and the like are melted. This improves adhesion of the resin (A), the thermoplastic resin (B), and the like to the screw, suppressing occurrence of vent flooding.

The number of the melting zones is preferably one or two, more preferably one. When the number of the melting zones is two or less, shear heating of the resin (A), the thermoplastic resin (B), and the like due to the screw or deformation compression of the resin (A), the thermoplastic resin (B), and the like is suppressed, thereby preventing excessive decomposition of the resin (A), the thermoplastic resin (B), and the like.

The barrel includes multiple barrel blocks connected in series.

The barrel block has a through hole corresponding to the cross-sectional shape of the screw.

A vacuum vent is provided for the purpose of removing low-boiling components in the resin (A), the thermoplastic resin (B), and the like upon melt-kneading of the resin (A), the thermoplastic resin (B), and the like using the screws of the twin-screw extruder.

The vacuum vent can be provided to the twin-screw extruder by using a barrel block with a vacuum vent, for example. The vacuum vent may be provided to multiple barrel blocks.

When only one material feed port is provided, the material feed port is disposed upstream of the most upstream kneading area.

When multiple material feed ports are provided, a first material feed port, which is the most upstream port among the raw material feed ports, is disposed upstream of the most upstream kneading area, while the other raw material feed port(s) may be disposed downstream of the most upstream kneading area. The resin (A) and the thermoplastic resin (B) are preferably fed through the first material feed port. Components other than the resin (A) and the thermoplastic resin (B) may be fed through the second or subsequent material feed ports.

When pelletizing the kneaded matter, the die used is preferably capable of extruding the kneaded matter into strands.

The number of discharging openings in the die may be one or more. The die preferably has several to several tens of discharging openings, which enables formation of multiple strands, leading to good productivity.
When melt-kneading is performed using the twin-screw extruder, first, the resin (A) and the thermoplastic resin (B) are charged into the raw material feed port of the twin-screw extruder. The resin (A) and the thermoplastic resin (B) fed through the raw material feed port of the twin-screw extruder are melt-kneaded in the twin-screw extruder.
The molten kneaded matter resulting from the melt-kneading in the twin-screw extruder is, for example, extruded through a die into strands. The strands are cut using, for example, a pelletizer. Thus, pellets of the thermoplastic resin composition are obtained.
The twin-screw extruder may be used for mixing in production of a masterbatch or for different mixing, and is preferably used at least for mixing in production of a masterbatch. The twin-screw extruder may be used in combination with a different mixer. For example, a masterbatch may be produced using the twin-screw extruder, and then the masterbatch may be mixed with a thermoplastic resin using a different mixer.
When the twin-screw extruder is used, the extrusion temperature is preferably the melting point of the thermoplastic resin (B) + 10°C or higher, more preferably the melting point + 20°C or higher, still more preferably the melting point + 30°C or higher, while preferably the melting point + 160°C or lower, more preferably the melting point + 150°C or lower, still more preferably the melting point + 130°C or lower.
In the method for producing a thermoplastic resin composition of the disclosure, the mixing step may be followed by an ejecting step of ejecting the mixture or a molding step of molding the ejected matter using a molding machine, for example.
Examples of the process in the molding step include, but are not limited to, extrusion molding, injection molding, and blow molding. To effectively achieve the molding processibility, extrusion molding is preferred among these.
The extrusion molding is performed using an extruder. Examples of the extruder include a single-screw extruder, a twin-screw extruder, and a tandem extruder. The extruder commonly includes a cylinder, a screw housed in the cylinder, a die attached to the tip of the cylinder, and a hopper for feeding pellets to the cylinder.
The conditions relating to the molding are not limited and may be set as appropriate in accordance with, for example, the compositional ratio and amount of the composition as well as the shape and size of a desired molded article.

<Molded article>

The molded article of the disclosure is obtainable from the thermoplastic resin composition of the disclosure and may be obtained by, for example, a molding step of molding the thermoplastic resin composition of the disclosure.
The disclosure also relates to a method for producing a molded article including a molding step.
The thermoplastic resin composition of the disclosure is suitable for a molded article in the form of a tube, a film, or a sheet, and may be applicable to a molded article in a different form.
The molding step is the same as that described for the method for producing a processing aid of the disclosure.
In the molding step, the molding temperature (extrusion temperature) during molding is commonly equal to or higher than the melting point of the thermoplastic resin (B) and lower than the decomposition temperature of the resin (A). To allow the processing aid to significantly exert its effects, the molding temperature preferably falls within a range of 160°C or higher and 270°C or lower.
The molding temperature may also be referred to the extrusion temperature in the case of extrusion molding.
Examples of applications of the molded article of the disclosure include, but are not limited to, bags, coating materials, dishware such as drink containers, electric wires, cables, pipes, fibers, bottles, gasoline tanks, and other industrial molded articles.

<Method for evaluating thermoplastic resin composition>

The method for evaluating a thermoplastic resin composition of the disclosure includes: a melting step of melting a thermoplastic resin composition containing a resin (A) and a thermoplastic resin (B) by heating at a temperature increase rate of 5°C to 15°C/min to 180°C to 200°C, followed by standing for 3 to 10 minutes; and an observing step of observing the melted thermoplastic resin composition under a polarizing microscope to evaluate the dispersed particle size of the resin (A).
With the evaluation method of the disclosure, the dispersed particle size of the resin can be determined with high accuracy.
The temperature increase rate is preferably 8°C to 14°C/min, more preferably 10°C to 12°C/min, still more preferably 10°C/min.
The final temperature during the heating is preferably 185°C to 195°C, more preferably 190°C/min.
The standing time after the heating is preferably four to eight minutes, more preferably four to six minutes, still more preferably five minutes.

In the evaluation method of the disclosure, the thickness of the thermoplastic resin composition to be evaluated is preferably 50 μm or less, more preferably 40 μm or less, still more preferably 30 μm or less. With such a thickness, the thermoplastic resin composition can be uniformly heated. The lower limit of the thickness is normally, but is not limited to, 5 μm or more.
It should be appreciated that a variety of modifications and changes in the structure and other details may be made to the aforementioned embodiments without departing from the spirit and scope of the claims.

EXAMPLES

The disclosure is described in more detail below with reference to examples, but is not limited to these examples.
The following materials were used in the examples and the comparative examples.

(Resin (A))

EVOH: ethylene-vinyl alcohol copolymer (ethylene content: 38 mol%, MFR: 1.6 g/10 min, melting point: 172°C)
PLA: polylactic acid (MFR: 3 g/10 min, melting point: 153°C)

(Thermoplastic resin (B) (carrier resin))

m-LLDPE-2: metallocene-catalyzed linear low-density polyethylene (MFR: 2.0 g/10 min, melting point: 121°C, d = 0.925, MIR = 23.6)
ZN-LLDPE-2: Ziegler-Natta-catalyzed linear low-density polyethylene (MFR: 2.0 g/10 min, melting point: 121°C, d = 0.918, MIR = 23.2)

(Thermoplastic resin (C) (matrix resin))

m-LLDPE-1: metallocene-catalyzed linear low-density polyethylene (MFR: 0.7 g/10 min, melting point: 123°C, d = 0.926, MIR = 28.1)
ZN-LLDPE-1: Ziegler-Natta-catalyzed linear low-density polyethylene (MFR: 0.8 g/10 min, melting point: 123°C, d = 0.925, MIR = 27.0)

Comparative Example 1, Examples 1 to 19

First, the carrier resin (thermoplastic resin (B)) and the resin (A) were melt-kneaded in the proportions shown in Table 1 or 2 using a twin-screw extruder (TEX25αIII available from Japan Steel Works, Ltd.) under the conditions including a cylinder temperature of 180°C to 200°C, a die temperature of 200°C, and a screw rotational speed of 300 rpm, whereby a masterbatch (MB) was obtained.
The twin-screw extruder used in the melt-kneading is more specifically described. FIG. 1 is a schematic structural view showing a twin-screw extruder used in the melt-kneading.
A twin-screw extruder 10 has, for example, two screws (not shown), a barrel 12 housing the two screws, and a material feed port 16 on the barrel 12, and a strand die head 18 at the downstream end of the barrel 12.
The barrel 12 includes a first barrel block C1 to a fifteenth barrel block C15 sequentially from the upstream side.
The material feed port 16 is provided on the first barrel block C1.
The twin-screw extruder 10 includes a melting zone (not shown) extending from a part of the sixth barrel block C6 to a part of the thirteenth block C13.
The screw elements other than the elements corresponding to the melting zone are all rotary elements. The melting zone is constituted by two or more kneading disk elements.
The number of kneading disk elements was set such that the kneading area ratio would reach the value shown in Table 1 or 2.
The dispersed particle size of the resin (A) in the resulting masterbatch was evaluated by the following dispersion evaluation. The result is shown in Table 1 or 2.
The masterbatch obtained was then dry blended with the matrix resin (thermoplastic resin (C)) in the proportion

that ensured the concentration of the resin (A) in the resulting thermoplastic resin composition remained constant (2000 ppm), and the processibility in this case was evaluated as in <Extrusion evaluation> described below. The result is shown in Table 1 or 2.

The resin (A), the thermoplastic resin (C), and the thermoplastic resin (B) each contained no fluorine and the masterbatch and the thermoplastic resin composition produced therefore had a fluorine content of 0% by mass.

<Dispersion evaluation>

(1) The masterbatch in which the resin (A) is dispersed in the thermoplastic resin (B) is cut in a direction perpendicular to the extrusion direction using a microtome, whereby 10 samples with a thickness of about 30 μm are obtained. The resulting samples are each heated at a temperature increase rate of 10°C/min to 190°C and then allowed to stand for 10 minutes. Thus, the samples are melted.

(2) The melted thermoplastic resin composition is observed using a polarizing microscope (Nikon ECLIPSE LV100N POL (camera: Nikon DS-Fi2), magnification: 200X). Images are taken and analyzed using analysis software (NIS-Elements D) to measure the particle size. One image is taken for each sample, and 10 images in total are taken. The average of the particle sizes of the dispersed particles in the images are used as the dispersed particle size. When the particles are not in the circular form, the major axis is used as the particle size.

<Extrusion evaluation>

Each material was extruded for 60 minutes through a single screw extruder (Rheomex OS available from HAAKE, L/D: 33, screw diameter: 20 mm, die diameter: 2 mm) under the conditions including a cylinder temperature of 170°C to 200°C, a die temperature of 200°C, and a shear rate of 450/sec, which was evaluated for the following items.

Before each test operation, linear low-density polyethylene containing 15% by mass of silica was fed into the hopper and the screw rotational speed was increased to 150 rpm to perform purging for about 15 minutes. The same matrix resin as one to be used for the test was then fed to perform purging for about 15 minutes. The screw rotational speed was returned to 30 rpm and extrusion was performed until the temperature stabilized. After the pressure surely returned to the initial pressure, a next experiment was performed. If the pressure did not return to the initial value, the above purging process was repeated until the pressure returned to the initial value and a next experiment was then performed.

(Melt fracture (MF) disappearance time)

The matrix resin alone was extruded until the pressure stabilized while melt fracture occurred on the entire surface. The point of time at which the screw appeared was defined as zero, and the matrix resin was then extruded for 60 minutes. In the examples where the processing aid or the masterbatch was used, they were fed to the hopper at the zero point. The appearance of a strand at an early stage of extrusion and the appearance of a strand at completion of extrusion were checked by visual and tactile inspection. The period of time was measured from the zero point of time described above to the point when MF disappeared. The shorter the period of time is, the better the MF disappearance time is. The cases where MF did not disappear are expressed with the symbol "-".

(Die buildup (DBU))

For the sample in which MF completely disappeared, extrusion evaluation was performed by long-run molding (three hours). The state of the die after extrusion was visually observed and the presence of DBU (die drool) was evaluated.

The DBU was evaluated on a 5-point scale from 1 to 5. The smaller the value is, the smaller the amount of DBU occurred and the better the evaluation is, with 1 indicating no occurrence of DBU.

[Table 1]

| Examples/ Comparative Example | Thermoplastic resin (C) | Composition of MB | | | | Concentration of additive (ppm) (A/(C + B + A)) | Extrusion evaluation | | Kneading area ratio | [Kneading area ratio] × [Concentration of resin (A) in MB] |
|---|---|---|---|---|---|---|---|---|---|---|
| | | Thermoplastic resin (B) | Resin (A) | Concentration of resin (A) in MB (% by mass) | Dispersed particle size of resin (A) in MB (μm) | | MF disappearance time (min) | DBU (5-point scale) | | |
| Comparative Example 1 | m-LLDPE-1 | - | - | - | - | - | - | 5 | 0.22 | - |
| Example 1 | ↑ | m-LLDPE-2 | EVOH | 50 | 100 | 2000 | 20 | 5 | 0.09 | 4.5 |
| Example 2 | ↑ | ↑ | ↑ | 40 | 70 | 2000 | 20 | 4 | 0.10 | 4.0 |
| Example 3 | ↑ | ↑ | ↑ | 30 | 38 | 2000 | 30 | 2 | 0.10 | 3.0 |
| Example 4 | ↑ | ↑ | ↑ | 20 | 35 | 2000 | 30 | 2 | 0.10 | 2.0 |
| Example 5 | ↑ | ↑ | ↑ | 10 | 32 | 2000 | 30 | 2 | 0.10 | 1.0 |
| Example 6 | ↑ | ↑ | ↑ | 30 | 20.2 | 2000 | 40 | 1 | 0.15 | 4.5 |
| Example 7 | ↑ | ↑ | ↑ | 50 | 37 | 2000 | 30 | 2 | 0.22 | 11.0 |
| Example 8 | ↑ | ↑ | ↑ | 40 | 31 | 2000 | 30 | 2 | 0.22 | 8.8 |
| Example 9 | ↑ | ↑ | ↑ | 30 | 15.1 | 2000 | 40 | 1 | 0.22 | 6.6 |
| Example 10 | ↑ | ↑ | ↑ | 20 | 12.3 | 2000 | 50 | 1 | 0.22 | 4.4 |
| Example 11 | ↑ | ↑ | ↑ | 15 | 6 | 2000 | 60 | 1 | 0.22 | 3.3 |
| Example 12 | m-LLDPE-1 | m-LLDPE-2 | PLA | 30 | 32.1 | 2000 | 30 | 2 | 0.10 | 3.0 |
| Example 13 | ↑ | ↑ | ↑ | 50 | 33.2 | 2000 | 30 | 2 | 0.22 | 11.0 |
| Example 14 | ↑ | ↑ | ↑ | 30 | 13.9 | 2000 | 50 | 1 | 0.22 | 6.6 |
| Example 15 | ↑ | ↑ | ↑ | 20 | 10.1 | 2000 | 60 | 1 | 0.22 | 4.4 |
| Example 16 | ↑ | ↑ | ↑ | 15 | 6 | 2000 | 60 | 1 | 0.22 | 3.3 |

[Table 2]

| Example/ Comparative Example | Thermoplastic resin (C) | Composition of MB | | | | Concentration of additive (ppm) (A/(C + B + A)) | Extrusion evaluation | | Kneading area ratio | [Kneading area ratio] × [Concentration of resin (A) in MB] |
|---|---|---|---|---|---|---|---|---|---|---|
| | | Thermoplastic resin (B) | Resin (A) | Concentration of resin (A) in MB (% by mass) | Dispersed particle size of resin (A) in MB (μm) | | MF disappearance time (min) | DBU (5-point scale) | | |
| Example 10 | m-LLDPE-1 | m-LLDPE-2 | EVOH | 20 | 12.3 | 2000 | 50 | 1 | 0.22 | 4.4 |
| Example 17 | ↑ ZN-LLDPE-1 | ZN-LLDPE-2 | ↑ | ↑ | 13.1 | 2000 | 50 | 1 | 0.22 | 4.4 |
| Example 18 | | m-LLDPE-2 | ↑ | ↑ | 12.9 | 2000 | 50 | 1 | 0.22 | 4.4 |
| Example 19 | ↑ | ZN-LLDPE-2 | ↑ | ↑ | 13.0 | 2000 | 50 | 1 | 0.22 | 4.4 |

REFERENCE SIGNS LIST

10: Twin-screw extruder

12: Barrel

16: Material feed port

18: Strand die head

C1 to C15: First barrel block to Fifteenth barrel block

## Claims

1. A thermoplastic resin composition comprising:

   a resin (A); and
   a thermoplastic resin (B),
   the resin (A) containing a structural unit represented by the following formula 1 and having a dispersed particle size of 1 to 100 μm:

   $$-X-(CR^1R^2)_n-Y-(CR^3R^4)_m-Z- \quad \text{(formula 1)}$$

   wherein X is a single bond or a divalent group optionally containing a functional group;
   Y and Z are each independently a group constituted by at least one selected from the group consisting of a single bond, -O-, -C(=O)-, -C(=O)O-, -OC(=O)O-, -C(=NR')-, - C(=NR')O-, -OC(=NR')O-, -S-, -S(=O)-, -S(=O)O-, -OS(=O)O-, $-S(=O)_2-$, $-S(=O))_2O-$, $-OS(=O))_2O-$, -P(=O)-, -P(=O)O-, - OP(=O)O-, $-P(=O)_2-$, $-P(=O)_2O-$, $-OP(=O)_2O-$, -NR'-, and - C(OR')R'-, wherein R's at respective occurrences are each independently a hydrogen atom or a C1-C10 hydrocarbon group;
   $R^1$, $R^2$, $R^3$, and $R^4$ are each independently a hydrogen atom or a C1-C10 hydrocarbon group; and
   n and m are each independently an integer of 0 to 10,
   with at least one of X, Y, or Z being -C(=O)-, - C(=O)O-, -OC(=O)O-, or -C(OR')R'-.

2. The thermoplastic resin composition according to claim 1,
   wherein the thermoplastic resin composition is substantially free from fluorine.

3. The thermoplastic resin composition according to claim 1 or 2,

   wherein, in the formula 1, X is a divalent group constituted by at least one selected from the group consisting of $X^1$ and $X^2$,
   $X^1$ is a group constituted by at least one selected from the group consisting of -C(=O)-, -C(=NR')-, $-S(=O)_2-$, -NR'-, -CR'R'-, and -C(OR')R'-, wherein R's at respective occurrences are each independently a hydrogen atom or a C1-C10 hydrocarbon group; and
   $X^2$ is a C1-C12 aromatic hydrocarbon group optionally containing a substituent.

4. The thermoplastic resin composition according to claim 3,
   wherein, in the formula 1, X is a divalent group containing at least one selected from the group consisting of -C(=O)-, -CR'R'-, and -C(OR')R'-, wherein R's at respective occurrences are each independently a hydrogen atom or a C1-C10 hydrocarbon group.

5. The thermoplastic resin composition according to any one of claims 1 to 4,
   wherein, in the formula 1, Y and Z are each independently a group constituted by at least one selected from the group consisting of a single bond, -O-, -C(=O)-, - C(=O)O-, -C(=NR')-, -C(=NR')O-, -S-, $-S(=O)_2-$, $-S(=O)_2O-$, - NR'-, and -C(OR')R'-, wherein R's at respective occurrences are each independently a hydrogen atom or a C1-C10 hydrocarbon group.

**6.** The thermoplastic resin composition according to claim 5,
wherein, in the formula 1, Y and Z are each independently a group constituted by at least one selected from the group consisting of a single bond, -O-, -C(=O)-, and -C(=O)O-.

**7.** The thermoplastic resin composition according to any one of claims 1 to 6,
wherein the resin (A) includes at least one selected from the group consisting of an ethylene-vinyl alcohol copolymer and polylactic acid.

**8.** The thermoplastic resin composition according to any one of claims 1 to 7,
wherein the resin (A) has a dispersed particle size of 5 to 100 μm.

**9.** The thermoplastic resin composition according to any one of claims 1 to 8,
wherein the thermoplastic resin (B) includes a polyolefin resin.

**10.** The thermoplastic resin composition according to any one of claims 1 or 9,
wherein the thermoplastic resin (B) includes metallocene-catalyzed linear low-density polyethylene.

**11.** The thermoplastic resin composition according to any one of claims 1 to 10,

wherein the thermoplastic resin composition includes a masterbatch, and
the resin (A) is contained in an amount of 8 to 50% by mass.

**12.** The thermoplastic resin composition according to any one of claims 1 to 10,

wherein the thermoplastic resin composition includes: a masterbatch containing the resin (A) and the thermoplastic resin (B); and a thermoplastic resin (C), and
the resin (A) is contained in an amount of 0.1 to 1.0% by mass.

**13.** The thermoplastic resin composition according to claim 12,
wherein the thermoplastic resin (C) includes metallocene-catalyzed linear low-density polyethylene.

**14.** A molded article comprising the thermoplastic resin composition according to any one of claims 1 to 13.

**15.** The molded article according to claim 14,
wherein the molded article is in a form of a tube, a film, or a sheet.

**16.** A method for producing the thermoplastic resin composition according to any one of claims 1 to 13, comprising a mixing step of mixing the resin (A) and the thermoplastic resin (B) using a twin-screw extruder.

**17.** The method for producing the thermoplastic resin composition according to claim 16,

wherein the twin-screw extruder includes a screw in which multiple screw elements, including two or more kneading disk elements, are mounted on a shaft and a barrel housing two of the screws, and
a kneading area ratio, a value obtained by dividing a total length of the kneading disk elements by a total length of the screw, is 0.01 or more.

**18.** A method for evaluating a thermoplastic resin composition, comprising:

a melting step of melting a thermoplastic resin composition containing a resin (A) and a thermoplastic resin (B) by heating at a temperature increase rate of 5°C to 15°C/min to 180°C to 200°C, followed by standing for 3 to 10 minutes; and
an observing step of observing the thermoplastic resin composition after melting under a polarizing microscope to evaluate a dispersed particle size of the resin (A).

FIG. 1

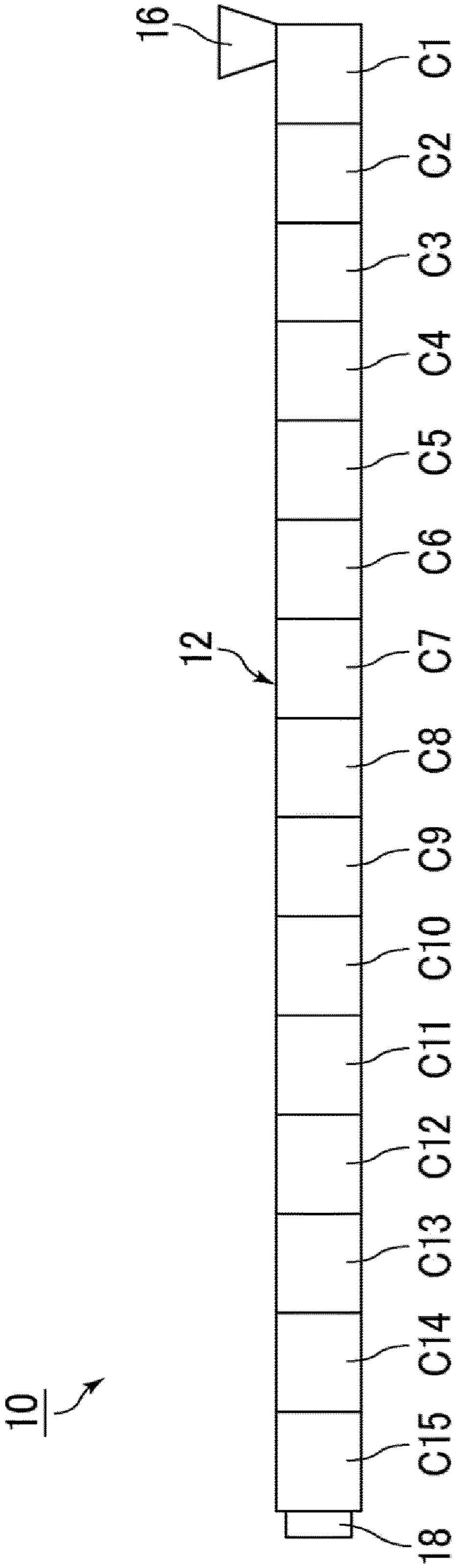

## INTERNATIONAL SEARCH REPORT

International application No.
**PCT/JP2024/046223**

**A. CLASSIFICATION OF SUBJECT MATTER**

***C08L 101/00***(2006.01)i; ***C08J 3/22***(2006.01)i; ***C08J 5/18***(2006.01)i; ***C08L 101/02***(2006.01)i
FI: C08L101/00; C08L101/02; C08J5/18; C08J3/22 CES

According to International Patent Classification (IPC) or to both national classification and IPC

**B. FIELDS SEARCHED**

Minimum documentation searched (classification system followed by classification symbols)

C08K3/00-13/08; C08L1/00-101/14; C08L101/16; C08J3/00-3/28;99/00;C08J5/00-5/02;5/12-5/22

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2018-141171 A (KURARAY CO., LTD.) 13 September 2018 (2018-09-13)<br>claims, example 2, comparative examples 1-2 | 1-10, 14-17<br>11-13 |
| X<br>A | JP 2009-155644 A (SUMITOMO CHEMICAL COMPANY, LIMITED) 16 July 2009 (2009-07-16)<br>claims, paragraphs [0047]-[0062], example 6 | 1-7, 9-11, 13-17<br>8, 12 |
| X<br>A | JP 2009-227833 A (TOPPAN PRINTING CO., LTD.) 08 October 2009 (2009-10-08)<br>claims, examples | 1-10, 14-17<br>11-13 |
| X<br>A | JP 2013-199532 A (SEKISUI PLASTICS CO., LTD.) 03 October 2013 (2013-10-03)<br>claims, comparative examples 1, 2 | 1-7, 9-10, 14-15<br>8, 11-13, 16-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 March 2025** | **25 March 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/046223**

**C. DOCUMENTS CONSIDERED TO BE RELEVANT**

| Category* | Citation of document, with indication, where appropriate, of the relevant passages | Relevant to claim No. |
|---|---|---|
| X | JP 2009-107316 A (TORAY INDUSTRIES, INC.) 21 May 2009 (2009-05-21) claims, comparative example 4 | 1-7, 9-10, 14-17 |
| A | | 8, 11-13 |
| A | JP 2014-34651 A (JSR CORPORATION) 24 February 2014 (2014-02-24) entire text | 1-17 |

Form PCT/ISA/210 (second sheet) (July 2022)

**INTERNATIONAL SEARCH REPORT**

International application No.
**PCT/JP2024/046223**

**Box No. III Observations where unity of invention is lacking (Continuation of item 3 of first sheet)**

This International Searching Authority found multiple inventions in this international application, as follows:

Document 1: JP 2018-141171 A (KURARAY CO., LTD.) 13 September 2018 (2018-09-13)
claims, examples (Family: none)

The claims are classified into the following two inventions.

(Invention 1)
Claims 1-17

Claim 1 has the technical feature of "a thermoplastic resin composition comprising a resin (A) and a thermoplastic resin (B), wherein the resin (A) comprises structural units represented by formula 1 and the resin (A) dispersion particle size of is 1-100 μm." However, in light of the content disclosed in document 1, said technical feature does not make a contribution over the prior art and thus cannot be said to be a special technical feature.

However, claim 10, which depends from claim 1, has the special technical feature of "the thermoplastic resin (B) being a metallocene catalyst-type linear low-density polyethylene." Claims 11-17 also share the same special technical feature with claim 10.

Thus, claims 1-17 are classified as invention 1.

(Invention 2)
Claim 18

The invention in claim 18 shares with claims 1-17, classified as invention 1, the common technical features of "a thermoplastic resin composition comprising a resin (A) and a thermoplastic resin (B)" and the "resin (A) dispersion particle size."

However, in light of the content disclosed in document 1, said technical features do not make a contribution over the prior art and thus cannot be said to be special technical features.

Claim 18 also does not depend from claims 1-17.

Claim 18 is not substantially identical to or similarly closely related to any of the claims classified as invention 1.

Thus, claim 18 cannot be classified as invention 1, and thus is classified as invention 2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-17**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/JP2024/046223**

| Patent document cited in search report | Publication date (day/month/year) | Patent family member(s) | Publication date (day/month/year) |
|---|---|---|---|
| JP 2018-141171 A | 13 September 2018 | (Family: none) | |
| JP 2009-155644 A | 16 July 2009 | US 2010/0261846 A1<br>claims, paragraphs [0065]-[0084], example 6 | |
| JP 2009-227833 A | 08 October 2009 | (Family: none) | |
| JP 2013-199532 A | 03 October 2013 | (Family: none) | |
| JP 2009-107316 A | 21 May 2009 | (Family: none) | |
| JP 2014-34651 A | 24 February 2014 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H01215840 A
- US 20230031000 A1


### Non-patent literature cited in the description


- Chemische Technik. Winnacker/Kuchler. Wiley-VCH, 2005, vol. 5
- **J. RYAN et al.** *Journal of Vinyl & Additive Technology*, March 2000, vol. 6 (1), 7-19